Europäisches Patentamt

European Patent Office

Office européen des brevets

(19)

(11) Veröffentlichungsnummer: **0 205 967 B1**

## (12) EUROPÄISCHE PATENTSCHRIFT

(45) Veröffentlichungstag der Patentschrift: **29.05.91**

(51) Int. Cl.⁵: **C12N 9/06**, C12Q 1/26

(21) Anmeldenummer: 86107203.1

(22) Anmeldetag: 27.05.86

(54) H2O2-bildende Sarcosinoxidase, ihre Herstellung und Verwendung.

(30) Priorität: 29.05.85 DE 3519218

(43) Veröffentlichungstag der Anmeldung:
30.12.86 Patentblatt 86/52

(45) Bekanntmachung des Hinweises auf die
Patenterteilung:
29.05.91 Patentblatt 91/22

(84) Benannte Vertragsstaaten:
AT BE CH DE FR GB IT LI LU NL SE

(56) Entgegenhaltungen:
EP-A- 0 135 070
DE-A- 3 600 563
GB-A- 2 005 689

CHEMICAL ABSTRACTS, Band 93, Nr. 7, 18.
August 1980, Seite 735, Zusammenfassung
Nr. 68683a, Columbus, Ohio, US; & JP-A-80
34 001 (NODA INSTITUTE FOR SCIENTIFIC
RESEARCH) 10-03-1980

(73) Patentinhaber: **BOEHRINGER MANNHEIM
GMBH**
**Patentabteilung, Abt. E Sandhofer Strasse
112-132 Postfach 31 01 20
W-6800 Mannheim 31 Waldhof(DE)**

(72) Erfinder: **Mayr, Ulrich, Dr. rer. nat.
Pürstlingstrasse 21
W-8200 Rosenheim(DE)**
Erfinder: **Möllering, Hans
Herrestrasse 10
W-8132 Tutzing(DE)**
Erfinder: **Siedel, Joachim, Dr. rer. nat.
Bahnhofstrasse 6
W-8131 Bernried(DE)**
Erfinder: **Seidel, Hans, Dr. rer. nat.
Waxensteinstrasse 6
W-8132 Tutzing(DE)**

(74) Vertreter: **Weickmann, Heinrich, Dipl.-Ing. et
al**
**Patentanwälte Dipl.-Ing. H.Weickmann
Dipl.-Phys.Dr. K.Fincke Dipl.-Ing.
F.A.Weickmann Dipl.-Chem. B. Huber Dr.-Ing.
H. Liska Dipl.-Phys.Dr. J. Prechtel Postfach
860820
W-8000 München 86(DE)**

CHEMICAL ABSTRACTS, Band 78, Nr. 17, 30. April 1973, Seite 223, Zusammenfassung Nr. 108034p, Columbus, Ohio, US; A. GRAZIANA-SANTIER: "Metabolism of C1 units. Enzymic characteristics of Penicillium roqueforti mycelia obtained with sarcosine as the nitrogen source"

EP 0 205 967 B1

**Beschreibung**

Die Erfindung betrifft eine neue Sarcosinoxidase mit im Vergleich zu bekannten Sarcosin-Oxidasen verbesserter Stabilität (Haltbarkeit), insbesondere in detergenshaltigen Analysenreagenzien.

Sarcosinoxidasen (E.C. 1.5.3.1.) sind unter anderem für die enzymatische Bestimmung von Sarcosin, Creatin und Creatinin verwendbar. Besonders die enzymatische Bestimmung von Creatinin in Serum, Plasma oder Urin gewinnt in der klinischen Diagnostik an Bedeutung. Durch Kopplung der von Creatininamidohydrolase (E.C. 3.5.2.10), Creatinamidinohydrolase (E.C. 3.5.3.3) und Sarcosinoxidase katalysierten Reaktionen wird letztlich aus Creatinin im stöchiometrischen Verhältnis von 1:1 $H_2O_2$ gebildet, welches sich auf einfache Weise colorimetrisch bestimmen läßt; als besonders geeignet haben sich für diese colorimetrische Bestimmung zahlreiche chromogene Systeme, z. B. solche vom "Trinder-Typ" (s. Bergmeyer "Methoden der enzymatischen Analyse", 4. Auflage, Band 1, (1983) Seite 197) erwiesen, bei denen in Gegenwart von Peroxidase aus 4-Aminoantipyrin und einem phenolischen oder anilinischen Kuppler durch $H_2O_2$ oxidativ ein Farbstoff erzeugt wird, dessen Menge (bzw. Intensität) in linearem Verhältnis zur gebildeten $H_2O_2$-Menge steht.

Ein solcher auf der Sarcosinoxidase-Reaktion beruhender Creatinin-Nachweis bietet gegenüber bekannten enzymatischen Creatinin-Tests (Bergmeyer, "Methoden der enzymatischen Analyse", 3. Auflage, Band II (1974) Seite 1834, Tanganelli et al, Clin. Chem. 28 (1983), Seite 1461) den Vorteil einer - je nach Art des verwendeten Farbkupplers - deutlich höheren Nachweisempfindlichkeit, was gerade wegen der niedrigen Serum-Creatinin-Konzentration im diagnostischen Entscheidungsbereich (44 - 97 $\mu$mol/l) von großer Bedeutung für die Analysengenauigkeit ist. Daneben ist auch die Haltbarkeit der chromogenen Substanzen im angewendeten neutralen, wäßrigen Medium besser als die von NADH, dem Indikator der UV-Tests. Im Vergleich zur auch heute noch am häufigsten eingesetzten Routinemethode, der Creatininbestimmung nach Jaffé (Hoppe Seyler's Z. Physiol. Chem. 10 (1886) 391) bietet dieses Verfahren außerdem eine wesentlich höhere Spezifität und damit eine verbesserte diagnostische Aussagekraft; schließlich wird damit auch der Umgang mit ätzenden, stark alkalischen Reagenzien vermieden.

Die Verwendung der Sarcosinoxidase z. B. zur Creatininbestimmung setzt allerdings voraus, daß sie im gebrauchsfertigen Reagenz wenigstens über mehrere Tage bei 0 bis 25°C ausreichend lagerstabil ist und außerdem bei Durchführung der Messung auch bei erhöhten Temperaturen (30 bis 37°C) über die erforderliche Mindestreaktionszeit kein signifikanter Aktivitätsverlust auftritt. Da in der klinischen Chemie häufig trübe, triglyceridreiche Seren als Probenmaterial anfallen, enthalten enzymatische Analysenreagenzien bevorzugt auch noch sogenannte Aufklarungssysteme, meist bestehend aus Kombinationen von Lipasen mit nichtionischen Detergenzien (polyoxyethylierte Alkyl- oder Aralkylalkohole) und Salzen von Gallensäuren wie Na-Cholat als Lösungsvermittler, die die störungsfreie optische Messung selbst stark lipämischer Proben erlauben.

Um zum Beispiel die Creatininbestimmung auch in solchen trüben Proben durchführen zu können, ist es daher nötig, daß die Sarcosinoxidase unter den obengenannnten Lager- bzw. Reaktionsbedingungen ausreichend resistent gegenüber Denaturierung oder Inaktivierung durch Detergenzien ist.

Es ist weiterhin wünschenswert, daß die Sarcosinoxidase geeignete enzymatische Eigenschaften aufweist wie z. B. eine niedrige Michaelis-Konstante für Sarcosin und eine hohe maximale Umsatzrate, denn durch diese Eigenschaften wird die Reaktionszeit bei der enzymatischen Sarcosin-, Creatin- und Creatininbestimmung wesentlich mitbestimmt. Da die enzymatische Bestimmung beispielsweise von Creatinin auch bei höheren Temperaturen (37°C) und in Gegenwart von Detergenzien und Lösungsvermittlern durchführbar sein sollte, sind geeignete enzymatische Eigenschaften gerade bei diesen belastenden Umgebungsbedingungen von Bedeutung.

Entsprechende Untersuchungen zeigten, daß die bekannten Sarcosinoxidasen in derartigen detergenzhaltigen Analysenreagenzien keine den Anforderungen genügende Lagerstabilität und/oder Haltbarkeit bei erhöhten Reaktionstemperaturen aufweisen.

Es besteht daher Bedarf an einer Sarcosinoxidase, die die obengenannten Eigenschaften, insbesondere Stabilitätskriterien erfüllt.

Die Aufgabe wird erfindungsgemäß gelöst durch die Verwendung von Sarcosinoxidase aus Streptomycetaceae.

Dieses $H_2O_2$ bildende Enzym weist bei 25°C in 0,15 mol/l K-Phosphat pH 7,9 in Gegenwart oberflächenaktiver Substanzen nach 2 Tagen noch eine Aktivität von mindestens 40 % der Ausgangsaktivität auf. Bei 37°C in detergenzhaltigem Milieu besitzt es für Sarcosin einen $K_M$-Wert von 2 bis 4 mmol/l. Die $K_M$-Werte der bekannten und für diese Bestimmungen hinreichend stabilen Sarcosinoxidasen liegen hingegen bei diesen Bedingungen bei etwa 16 bis 20 mmol/l.

Das erfindungsgemäße Enzym findet sich bei allen Gattungen der Familie Streptomycetacea (The

3

Prokaryotes, Vol. II (1981), 2028), z. B. bei Chainia purpurogena DSM 43 156, Chainia ochraceae DSM 43 155, Streptomyces flocculus DSM 40 327, Streptoverticillium sp. DSM 40 237 und Kitasatoa purpurea DSM 43 362.

Das Enzym der Erfindung setzt sich aus vier verschiedenen Untereinheiten zusammen. Das Molekulargewicht beträgt etwa 170 kD.

Das Enzym ist stabil im pH-Bereich von 6 bis 9 und bei Temperaturen unter 40°C. Bei 50°C wird es in einem Zeitraum von 15 Minuten inaktiviert. Die optimale Temperatur der Reaktion liegt bei ca. 37°C, das pH-Optimum liegt um pH 8,0. Die hohe Substratspezifität zeigt sich an dem nur geringen Umsatz von Substratanalogen; so beträgt die Umsatzrate mit z. B. N,N-Dimethylglycin nur 1 % der Sarcosin-spezifischen Reaktion.

Die bei 25°C in verschiedenen Belastungsreagenzien gemessenen $K_M$-Werte für Sarcosin (Phosphatpuffer; TES-Puffer) liegen bei 2 bis 3 mmol/l. Die $V_{max}$ beträgt etwa 6 U/mg Protein. Nachstehende Tabelle I gibt für verschiedene Präparationen des erfindungsgemäßen Enzyms die bei 25 und bei 37°C gemessenen $K_M$-Werte für Sarcosin an. Zum Vergleich wurden die entsprechenden Werte für das bekannte Bacillus-Enzym angegeben.

EP 0 205 967 B1

### T a b e l l e   I

| Herkunft der Sarcosinoxidase | T(°C) | Puffer | $K_M$-Wert (mmol/l) für Sarcosin in Belastungsreagenz a |
|---|---|---|---|
| Chainia purpurogena (DSM 43156) Reinenzym | 25 | Phosphat | 2 |
| " | 37 | " | 3,5 |
| " | 25 | TES | 2,8 |
| " | 37 | " | 3 |
| Chainia purpurogena Rohextraktüberstand | 37 | Phosphat | 3 |
| " | 37 | TES | 3 |
| Chainia ochraceae (DSM 43 155) Rohextraktüberstand | 37 | Phosphat | 3,5 |
| " | 37 | TES | 3,6 |
| Streptomyces flocculus (DSM 40327) Rohextraktüberstand | 37 | TES | 3,5 |
| Bacillus sp. | 25 | Phosphat | 16 |
| " | 25 | TES | 20 |
| " | 37 | Phosphat | 18 |
| " | 37 | TES | 20 . |

Belastungsreagenz a ( /l):
0,15 mol K-Phosphat bzw. 0,1 mol TES/KOH, pH 7,9; 8,6 mmol 2,4,6-Tribrom-3-hydroxibenzoesäure, 0,8 mmol 4-Aminoantipyrin, 10 $\mu$mol $K_4(Fe(CN)_6)$, 5 mmol Na-Cholat, 0,5 % Lutensol ON 50, 0,2 % $NaN_3$, 0,5 mmol Titriplex III, 2000 U Lipase, 2000 U Peroxidase, 10000 U Ascorbatoxidase.

Das erfindungsgemäße Enzym zeichnet sich durch eine überlegene Stabilität in detergenzhaltigem Milieu bei 37° C aus, die sowohl in Rohextraktüberstand, als auch beim gereinigten Enzym vorliegt. Die Stabilität des erfindungsgemäßen Enzyms im Vergleich zu bekannten Sarcosinoxidasen zeigt die nachstehende Tabelle II.

5

EP 0 205 967 B1

$$T \quad a \quad b \quad e \quad l \quad l \quad e \quad \quad II$$

| Herkunft der Sarcosinoxidase | % Restaktivität nach Inkubation in Belastungsreagenz b bei 37°C über verschiedene Zeitintervalle | | | |
|---|---|---|---|---|
| | 15 min | 30 min | 45 min | 60 min |
| Pseudomonas maltophilia | 7 | 3 | 2 | 0 |
| Corynebacterium sp. U 96 | 6 | 2 | 1 | 0 |
| Bacillus sp. | 87 | 84 | 78 | 75 |
| Arthrobacter sp. | 7 | 1 | 0 | 0 |
| Cylindrocarpon didymum M-1 | 0 | 0 | 0 | 0 |
| Chainia purpurogena Reinenzym | 90 | 87 | 83 | 77 |

| Herkunft der Sarcosinoxidase | % Restaktivität nach Inkubation in Belastungsreagenz b bei 37°C über verschiedene Zeitintervalle | | | |
|---|---|---|---|---|
| Chainia purpurogena Rohextraktüberstand | 90 | 87 | 82 | 75 |
| Streptomyces flocculus Rohextraktüberstand | 97 | 97 | 96 | 94 |

Belastungsreagenz b ( I):

0,15 mol K-Phosphat pH 7,9; 8,6 mmol 2,4,6-Tribrom-3-hydroxibenzoesäure, 0,8 mmol 4-Aminoantipyrin, 10 $\mu$mol $K_4(Fe(CN)_6)$, 5 mmol Na-Cholat, 0,5 % Lutensol ON 50, 0,2 % $NaN_3$, 0,5 mmol Titriplex III, 2000 U Lipase, 2000 U Peroxidase, 10000 U Ascorbatoxidase, 25000 U Creatininase, 12000 U Creatinase, >100 U Sarcosin-Oxidase.

Die obigen Werte zeigen, daß nur das Enzym aus Bacillus eine vergleichbare Stabilität aufweist, während alle anderen Enzyme eine für die Praxis völlig ungenügende Stabilität besitzen.

In der nachstehenden Tabelle III wird die Langzeitstabilität bei 25°C des erfindungsgemäßen Enzyms und des Bacillus sp. Enzyms gezeigt.

6

EP 0 205 967 B1

T a b e l l e   III

| Herkunft der Sar-OD | Puffer | % Restaktivität nach zweitägiger Inkubation in Belastungsreagenz c bei 25°C |
|---|---|---|
| Chainia purp. Reinenzym | Phosphat TES | 83 80 |
| Chainia purp. Rohextrakt | Phosphat TES | 86 88 |
| Chainia ochr. Rohextrakt | Phosphat TES | 75 75 |
| Streptomyces flocc. Rohextrakt | Phosphat TES | 44 35 |
| Bac. sp. Reinenzym | Phosphat TES | 15 15 |

Belastungsreagenz c:
Zusammensetzung wie Belastungsreagenz b, jedoch ohne Zusatz des chromogenen Farbsystems. Neben 0,15 mol K-Phosphat pH 7,9 wurde auch 0,1 mol TES/KOH eingesetzt.

Die obigen Werte zeigen, daß das Enzym der Erfindung dem besten bisher bekannten Sarcosinoxidaseenzym hinsichtlich der Langzeitstabilität weit überlegen ist. Dies ist insbesondere wichtig für die Lagerbeständigkeit, die mit der Langzeitbeständigkeit korreliert.

Das erfindungsgemäße Enzym ermöglicht daher aufgrund seiner niedrigeren Michaelis-Konstante eine wesentlich schnellere Durchführung der enzymatischen Bestimmung von Sarcosin, Creatin oder Creatinin, ist bei 0 bis 25°C weitaus besser lagerungsbeständig und ist über den Inkubationsintervall bei Sarcosin-, Creatin- oder Creatininbestimmungen bei 37°C wesentlich stabiler als die meisten der bekannten Sarcosinoxidasen.

Die folgenden Beispiele erläutern die Erfindung weiter.

**Beispiel 1**

Anzucht von Chainia purpurogena DSM 43156

Der Organismus wurde in einem komplexen Medium folgender Zusammensetzung in einem Schüttelkolben kultiviert: 5 g Hefeextrakt, 3 g Pepton (tryptisch verdaut), 2 g NaCl, 0,24 g $MgSO_4$ x 7 $H_2O$, 0,014 g $CaCl_2$ x 7 $H_2O$, 2 g Glucose, 10 g Sarcosin, 1 l $H_2O$, pH 7,0. Die bei 28°C angezogenen Kulturen lieferten nach 30 Stunden eine Aktivität von ca. 400 U/l.

**Beispiel 2**

7

Isolierung der Sarcosinoxidase E.C. 1.5.3.1 aus Chainia

2,9 kg Feuchtmasse Chainia purpurogena DSM 43156 (resultierend aus 95 l Kultur) wurden mit 15 l 20 mmol/l Phosphatpuffer, pH 8,0 suspendiert und 4 Stunden bei 25° C mit 4 g Lysozym aufgeschlossen. Zu der Aufschlußsuspension wurde so viel 10%ige Polyethylenimin (PolyminG-20-)Lösung (BASF) zugegeben, daß eine weitgehende Abtrennung von Nucleinsäuren und Fremdproteinen erfolgte.

Die Sarcosinoxidase (befindet sich im Überstand) wurde an schwach basischen Anionenaustauscher (DEAE-Sephadex) gebunden und anschließend mit steigendem Salzgradienten eluiert. Das Eluat wurde mit Ammoniumsulfat auf eine Konzentration von 0,6 mol/l eingestellt, das Enzym an Phenyl-Sepharose gebunden und mit absteigendem Ammoniumsulfatgradienten (obiger Phosphatpuffer) chromatographiert. Die Eluate mit über 4 U/mg Protein wurden mit festem Ammoniumsulfat auf eine Konzentration bis 2,4 mol/l gebracht. Der Niederschlag wurde mit 0,1 mol/l Phosphatpuffer aufgenommen und die Sarcosinoxidase durch eine Molekularsieb-Passage (Sephacryl-S-200, Pharmacia) weiter gereinigt.

Das gereinigte Enzym hat eine spezifische Aktivität von 5,5 U/mg Protein.

## Beispiel 3

Anwendung der Sarcosin Oxidase für die Creatinin-Bestimmung

a) Reagenz I (Probenleerwertreagenz):

| | |
|---|---|
| K-Phosphat (pH 7,9) | 150 mmol/l |
| | (oder 0,1 mol/l TES/KOH pH 7,9) |
| 4-Aminoantipyrin | 0,8 mmol/l |
| 2,4,6-Tribrom-3-hydroxy-benzoesäure | 8,6 mmol/l |
| $K_4(Fe(CN)_6)$ | 10 µmol/l |
| Na-Cholat | 5 mmol/l |

| | |
|---|---|
| Lutensol ® ON 50 | 0,5 % (w/v) |
| Creatinamidinohydrolase | 12 U/ml |
| Sarcosin-Oxidase nach Beispiel 2 | 6,5 U/ml |
| Peroxidase | 2 U/ml |
| Lipase | 2 U/ml |
| Ascorbat-Oxidase | 10 U/ml |

b) Reagenz II (Probenreagenz):

| | |
|---|---|
| Reagenz I plus Creatinin-amidohydrolase | 25 U/ml |

c) Testdurchführung / Bestimmungsansatz:

Wellenlänge 546 nm; T = 25° C (oder 37° C);
Schichtdicke = 1 cm
Messung gegen Luft

| | ① Reagenz I - Leerwert | ② Probenleer- wert | ③ Reagenz II- Leerwert | ④ Proben- wert |
|---|---|---|---|---|
| Reagenz I | 1,00 ml | 1,00 ml | – | – |
| Reagenz II | – | – | 1,00 ml | 1,00 ml |
| $H_2O$ | 0,05 ml | – | 0,05 ml | – |
| Probe | – | 0,05 ml | – | 0,05 ml |

20 Minuten bei 25 oder 37°C inkubieren, dann Extinktionen $E_1 - E_4$ messen.

$$E = (E_④ - E_③) - (E_② - E_①).$$

Berechnung der Creatinin-Konzentration in der Probe:
Über mitgeführten wäßrigen Standard (2mg/dl). Der Standard ist hierzu im Bestimmungsansatz wie die Probe einzusetzen.

**Ansprüche**

1. $H_2O_2$-bildende Sarcosinoxidase,
   **dadurch gekennzeichnet,**
   daß sie aus Streptomycetaceae erhältlich ist und bei 25° C in 0,15 mol/l K-Phosphat pH 7,9 in Gegenwart oberflächenaktiver Substanzen nach 2 Tagen noch eine Aktivität von mindestens 40 % der Ausgangsaktivität aufweist.

2. Verwendung der Sarcosinoxidase von Anspruch 1 zur Bestimmung von Sarcosin, Creatin oder Creatinin.

Patentansprüche für folgenden Vertragsstaat: AT

1. Verfahren zur Gewinnung einer $H_2O_2$-bildenden Sarcosinoxidase,
   **dadurch gekennzeichnet,**
   daß man aus Biomasse von Streptomycetaceae eine Sarcosinoxidase, die bei 25° C in 0,15 mol/l K-Phosphat pH 7,9 in Gegenwart oberflächenaktiver Substanzen nach 2 Tagen noch eine Aktivität von mindestens 40 % der Ausgangsaktivität aufweist, isoliert.

2. Verwendung der Sarcosinoxidase von Anspruch 1 zur Bestimmung von Sarcosin, Creatin oder Creatinin.

### Claims

1. H₂O₂-forming sarcosine oxidase, characterised in that it is obtainable from Streptomycetaceae and at 25° C. in 0.15 mol/l. K phosphate pH 7.9, in the presence of surface-active substances, still shows after 2 days an activity of at least 40% of the initial activity.

2. Use of the sarcosine oxidase according to claim 1 for the determination of sarcosine, creatine or creatinine.

Claims for the following Contracting State: AT

1. Process for the obtaining of an H₂O₂-forming sarcosine oxidase, characterised in that one isolates from a biomass of Streptomycetaceae a sarcosine oxidase which, at 25° C. in 0.15 mol/l. K-phosphate pH 7.9, in the presence of surface-active substances, still displays an activity of at least 40% of the initial activity after 2 days.

2. Use of the sarcosine oxidase of claim 1 for the determination of sarcosine, creatine or creatinine.

### Revendications

1. Sarcosine oxydase formant du H₂O₂, caractérisée en ce qu'elle peut être obtenue à partir de Streptomycetaceae et présente à 25° C dans 0,15 mol/l de phosphate K pH 7,9 en présence de substances tensio-actives après deux jours encore une activité d'au moins 40 % de l'activité de départ.

2. Utilisation de la sarcosine oxydase de la revendication 1 pour la détermination de la sarcosine, de la créatine ou de la créatinine.

Revendications pour l'Etat contractant suivant: AT

1. Procédé pour l'obtention d'une sarcosine oxydase formant du H₂O₂, caractérisé en ce qu'on isole, à partir de la biomasse de Streptomycetaceae, une sarcosine oxydase qui présente à 25° C dans 0,15 mol/l de phosphate K pH 7,9 en présence de substances tensio-actives après deux jours encore une activité d'au moins 40 % de l'activité de départ.

2. Utilisation de la sarcosine oxydase de la revendication 1 pour la détermination de la sarcosine, de la créatine ou de la créatinine.